# EUROPEAN PATENT APPLICATION

(11) **EP 0 976 405 A1**
(43) Date of publication of application: **02.02.2000**
(21) Application number: 98914050.4
(22) Date of filing: 16.04.1998
(51) Int. Cl.: A61K 47/10

(54) **BASE COMPOSITION FOR PERCUTANEOUS ABSORPTION AND PERCUTANEOUSLY ABSORBABLE PREPARATION CONTAINING THE BASE COMPOSITION**

(30) Priority: 16.04.1997 JP 11525497
(71) Applicant: HISAMITSU PHARMACEUTICAL CO. INC., Tosu-shi Saga 841-0017 (JP)
(72) Inventor: HIRANO, Munehiko, Hisamitsu Pharm. Co., Inc., Tosu-shi Saga 841-0017 (JP); MAKI, Masayoshi, Hisamitsu Pharm.Co., Inc., Tosu-shi Saga 841-0017 (JP)
(74) Representative: Cresswell, Thomas Anthony
(86) International application number: JP9801745
(87) International publication number: WO9846267

(57) **Abstract**

A base composition for percutaneous absorption, which comprises a styrene/isoprene/styrene block copolymer, a softening agent, an adhesive resin and hexylene glycol; and a percutaneously absorbable preparation comprising the composition and a medicine.

## Description

### Technical Field of the Invention

This invention is concerned with a field of a percutaneous drug therapy and it relates to a percutaneously absorbable preparation, characterized in that, the said preparation uses a styrene-isoprene-styrene block copolymer, a softener, a tackifying resin and hexylene glycol as the ingredients for the substrate composition for percutaneous absorption whereby solubility and skin permeation of the pharmaceutical are made good and a predetermined amount of the pharmaceutical can be correctly and surely administered to patients.

### Background Art

Estradiol which is contained in follicular hormone is secreted from ovary during the period when female is capable of reproducing. Accordingly, the female before and after menopause mostly lacks in estradiol causing symptoms of menopausal disorder, menstrual irregularities, etc. In order to ameliorate such symptoms, a therapy by administration of oral preparations, etc. is carried out at present. However, the ingredient is quickly metabolized and inactivated by digestive organs such as stomach and intestine as well as liver or the like and, therefore, administration of estradiol of a high dose is necessary in order to expect an onset of sufficient pharmacological effect. In addition, there is a possibility that manifestation of side effects, etc. increases due to the high dose.

Under such circumstances, a percutaneous administration where metabolism of estradiol is made little whereby it is well delivered to blood and is subjected to the therapy has been attempted. On the other hand, an investigation where corpus luteum hormone which is another hormone is percutaneously absorbed to suppress the side effect by administration of estradiol has been carried out as well. In the Japanese Patent Laid-Open No. 342532/1992, there is a proposal for a percutaneously absorbable preparation in which estradiol and corpus luteum hormone are effective ingredients and an acrylic adhesive consisting of 2-ethylhexyl acrylate and N-vinyl-2-pyrrolidone is a main component as an adhesive. However, the acrylic adhesive has a low releasing ability, has a strong irritation to skin and is not durable for a continuous administration for a long period.

In the Japanese Patent Laid-Open No. 51623/1994, there is a proposal for a method where estradiol and norethisterone acetate which are pharmaceutical ingredients are dissolved in a gel consisting of hydroxypropyl cellulose and ethanol, the solution is made into a reservoir type, and release of the pharmaceutical ingredients is controlled using a membrane where the permeability is adjusted. However, there is a problem of side effect that ethanol has a strong irritation to skin and results in flare at the applied area in a high frequency. In the meanwhile, a percutaneous absorbable patch consisting of a styrene-isoprene-styrene block copolymer where crotamiton is used as a solubilizer is proposed in the International Laid-Open Patent Publication WO 91/17752 and the Japanese Patent Laid-Open No. 148145/1993. However, when crotamiton is used as a solubilizer, there is a problem in terms of stability that the styrene-isoprene-styrene block copolymer itself is dissolved in crotamiton and the expected cohesive force is not available.

Hexylene glycol (generic name; its chemical name is 2-methyl-2,4-pentanediol) is usually used as moisturizer, solvent, industrial cleaning agent, hydraulic fluid, softener for leather and fabrics, additive to ink, photographic additive, etc. In the Japanese Patent Laid-Open No. 109220/1995 and No. 53338/1996, agents for external use where hexylene glycol is used as an antibacterial agent are proposed.

Further, in the International Laid-Open Patent Publication WO 96/19976 and the Japanese Patent Laid-Open No. 138153/1995, agents for external use where hexylene glycol is used as an absorption accelerator are proposed. However, hexylene glycol has a high intermiscibility with an acrylic substrate and, in order to achieve a sufficient absorption accelerating effect, it is necessary to compound a large amount of hexylene glycol. There is another problem that compounding of large amount of hexylene glycol lowers the adhesion and affects the fundamental physical property of the preparation.

In view of the above-mentioned problems, the present inventors have continued an intensive investigation with an object of offering a percutaneous absorbable preparation or a substrate composition having:
1) improved solubility and stability of the pharmaceutical ingredients;
2) improved permeation of the pharmaceutical ingredients into skin;
3) low irritation to skin; and
4) stabilized physical property of the substrate and have accomplished this invention.

As a result of an intensive investigation for solving the above problems, the present inventors have found that, when styrene-isoprene-styrene block copolymer, softener, tackifying resin and hexylene glycol are used as the ingredients for the substrate composition, it is now possible to give a percutaneously absorbable substrate composition having good cohesive force, stabilized physical property and good solubility, stability and skin-permeability of the pharmaceutical whereupon this invention has been achieved.

### Disclosure of the Invention

This invention relates to a percutaneously absorbable substrate composition containing styrene-isoprene-styrene block copolymer, softener, tackifying resin and hexylene glycol in a sufficient amount for showing an accelerating action of percutaneous absorption or for dissolving the pharmaceutical.

This invention also relates to a percutaneously absorbable preparation containing a pharmaceutical and the said percutaneously absorbable substrate composition.

More particularly, this invention relates to a percutaneously absorbable substrate composition containing styrene-isoprene-styrene block copolymer, softener, tackifying resin and hexylene glycol as the ingredients for the substrate composition and also to a percutaneously absorbable patch containing the said percutaneously absorbable substrate composition and a pharmaceutical.

After the percutaneously absorbable patch of this invention is applied to the skin of the patient, the pharmaceutical in an therapeutically effective amount can be correctly and surely released and this invention is to offer a percutaneously absorbing patch.

### Brief Explanation of the Drawings

Fig. 1 shows a skin permeability of estradiol (E₂) in the preparations of Example 3 and Comparative Examples 2 and 3 using hairless mice.
Fig. 2 shows a skin permeability of norethisterone acetate (NETA) in the preparations of Example 3 and Comparative Examples 2 and 3 using hairless mice.
Fig. 3 shows the result of the releasing test of estradiol (E₂) in the preparations of Example 3 and Comparative Examples 1 and 6.
Fig. 4 shows the result of the releasing test of norethisterone acetate (NETA) in the preparations of Example 3 and Comparative Examples 1 and 6.

### Best Mode for Carrying Out the Invention

A pharmaceutical which is an effective ingredient in the percutaneously absorbable preparation of this invention is to be a physiologically active substance and is to have a percutaneously absorbing ability. Alternatively, it may be the so-called pro-drug which shows a physiological activity after being absorbed percutaneously. Further, it may be a pharmaceutically acceptable inorganic or organic addition salt.

Preferred examples of the pharmaceutical for the percutaneously absorbable preparation of this invention are female sex hormones such as follicular hormones, corpus luteum hormones and derivatives thereof. Examples of active ingredients in the case of follicular hormones are estradiol, estrone, estriol, equilin, equilenin and derivatives thereof. In the percutaneously absorbable preparation of this invention, estradiol is used preferably. In the case of corpus luteum hormones, the examples are progesterone, hydroxyprogesterone caproate, medroxyprogesterone acetate, dydrogesterone, chlormadinone acetate, ethisterone, dimethisterone, norethisterone, norethisterone acetate, norethisterone enanthate, ethynodiol acetate, megesterol acetate and allylestrenol and, preferably, norethisterone acetate is mostly used in the percutaneously absorbable preparation of this invention.

Besides the above, examples of the pharmaceutical which is effective in the percutaneously absorbable preparation of this invention are antiemetic agents (such as granisetron, azasetron, ondansetron and ramosetron), therapeutic agents for pollakisuria (such as oxybutynin), inhibitors for angiotensin I converting enzymes (such as captopril and delapril), calcium antagonists (such as nifedipine), coronary vasodilators (such as diltiazem and nicorandil), local anesthetics (such as lidocaine and procaine), thymus hormones (such as serum thymus factor), muscle relaxants (such as tizanidine, eperisone and dantrolene), stimulants, antihypertensive agents (such as alprenolol and nifedipine), anti-tumor agents, psychotropic agents (such as imipramine, fentanyl and morphine), antibiotic substances, anti-parkinsonism agents (such as amantadine and levodopa), antihistaminic agents, anti-vertigo agents (difenidol and betahistine), hypnotic analgesics, anti-inflammatory analgesics (such as indomethacin, ketoprofen, diclofenac, ibuprofen, flurbiprofen, felbinac and ketorolac), autonomic agents, pharmaceuticals for heart and blood vessels (such as benzothiazepine), antitussive expectorants (such as ketotifen, tulobutelol and tranilast), agents for improvement of cerebral circulation and metabolism (such as vinpocetine), vitamins, hormones of a polypeptide type (such as luteinizing hormone-releasing hormones and thyrotropin-releasing hormones), peripheral blood vessel dilators, immunomodulators (such as polysaccharides, auranofin and lobenzarit), chologogues (such as ursodesoxycholic acid), diuretics (such as hydroflumethiazide), anti-diabetic agents (such as tolbutamide), therapeutic agents for gout (such as colchicine), and the like. Therapeutically effective amount thereof varies depending upon the object of compounding but, usually, a compounding amount of 0.1-10% by weight is preferably used. Two or more of those pharmaceuticals may be used jointly if necessary in case there is no inconvenience due to their interaction.

A combination of styrene-isoprene-styrene blockcopolymer, softener, tackifying resin and hexylene glycol in the percutaneously absorbable substrate composition of this invention is able to contain a pharmaceutical in such a large amount that cannot be predicted from the conventional rubber type substrate compositions and, in addition, it is possible to release such a high amount of a pharmaceutical that is nor available by the sole use of an acrylic type one.

Further, hexylene glycol can be used within such a range that it does not substantially dissolve the ingredients of the substrate composition, particularly styrene-isoprene-styrene block copolymer or that a substantial dissolution is not noted and, therefore, good cohesive force and stability can be achieved.

Contents of those ingredients to the total amount of the preparation are as follows.

Thus, 10-30% by weight, preferably 13-27% by weight or, more preferably, 15-25% by weight for the styrene-isoprene-styrene block copolymer; 10-60% by weight, preferably 12-55% by weight or, more preferably, 15-50% by weight for the softener; and 20-60% by weight, preferably 23-57% by weight or, more preferably, 25-50% by weight for the tackifying resin. A combination within the above range is most effective in achieving the merit of this invention.

When the content of the styrene-isoprene-styrene block copolymer is less than the above range, cohesive force is insufficient while, when it is more than that, flexibility of the preparation is little resulting in a problem in adhesion. When the content of the softener is less than the above, flexibility of the preparation is little resulting in a problem in adhesion while, when it is more than that, flexibility becomes big but there is a problem in cohesive force of the preparation. Tackifying resin has an appropriate tackiness and an intermiscibility with hexylene glycol within the above-mentioned range. If the tackifying resin is outside of the above range, sufficient compounding of hexylene glycol and a sufficient absorption accelerating effect by hexylene glycol are not achieved.

Hexylene glycol which is one of the ingredients of this invention has been known to be used as a moisturizer and an antibacterial agent as cosmetic materials but, in this invention, hexylene glycol is to be compounded in a sufficient amount as an absorption accelerating agent and/or a solubilizer for a pharmaceutical ingredient and its compounding amount is 1-10% by weight, preferably 1.5-8% by weight or, more preferably, 2-7% by weight. When the compounding amount is less than 1% by weight, its effect for stabilization of the substrate composition and for solubility and absorption acceleration of the pharmaceutical ingredient are insufficient while, when it is more than 10% by weight, bleeding by hexylene glycol is resulted.

Hexylene glycol which is compounded with the percutaneously absorbable substrate composition of this invention can be used together with other absorption accelerating agent and/or solubilizer although a sole use of hexylene glycol without other absorption accelerator or solubilizer is preferred.

With regard to the percutaneously absorbable preparation of this invention, patch is preferred and the percutaneously absorbable patch of this invention may be prepared in such a manner that a pharmaceutical-containing layer consisting of the pharmaceutical and the percutaneously absorbable substrate composition is formed substantially as a single layer but, if there is no influence on the absorption of the pharmaceutical, other layer may be further formed to give a multi-layered product.

With regard to a dosage form of the percutaneously absorbable patch of this invention, hard plaster is preferred and substantially anhydrous hard plaster is particularly preferred.

Examples of the styrene-isoprene-styrene block copolymer are those manufactured by Shell Chemical (trade names: Cariflex TR-1107 and Cariflex TR-1111), those manufactured by Japan Synthetic Rubber (trade names: JSR 5000 and JSR 5100) and that manufactured by Nippon Zeon (trade name: Quintac 3421).

Examples of the softener are liquid paraffin, polybutene, castor oil, cotton seed oil, palm oil, coconut oil and process oil.

Examples of the tackifying resin are alicyclic saturated hydrocarbon resins (such as Arkon P-100 (trade name)), rosin esters (such as KE-311 and KE-100 (trade names); and Super Ester S-100 (trade name)), hydrogen alicyclic hydrocarbons (such as Escorez 5300 (trade name)), hydrogenated resins of a terpene type (such as Clearon P-105 (trade name)) and hydrogenated rosin esters (such as Foral 105 (trade name)).

Then, a film which is a support in this invention is to have a property of an excellent so-called barrier property for prevention of leakage, evaporation and adsorption of the pharmaceutical. It is also preferred that the film has an appropriate flexibility when applied onto the skin. There is. no particular limitation for the material of the support so far as the above-mentioned requirements are satisfied and its specific examples are aluminum, ethylene-vinyl acetate copolymer or a saponified product thereof, cellulose acetate, cellulose, Nylon, polyester, polyethylene, polyvinylidene chloride, polycarbonate, polyvinyl alcohol and polypropylene. Such a material may be made into filmy or, if necessary, it is made into a form of paper or cloth followed by laminating with a film to give a layered film or subjected to a vapor deposition with aluminum or ceramic so that a barrier property can be improved.

With regard to a film which is to be a releasable liner layer, it is necessary to inhibit leakage, evaporation, etc. from a pharmaceutical layer during the preservation of the preparation and, in addition, the said releasable liner layer is able to be released and removed when the preparation is used. Specific examples of the material for the releasable liner film which can be used are aluminum, cellulose, polyester, polyethylene and polypropylene and, if necessary, such a film may be layered. Further, its surface may be treated with silicone or fluorocarbon or a known additive may be compounded in the liner material for adjusting the releasing ability or the barrier property. It is also possible to make a pinch for releasing on a releasable liner whereby a handling upon releasing is easy.

Furthermore, for adjustment of adhesion, safety and stability, known additives may be compounded if necessary. For example, appropriate amounts of tackifiers or adhesives (such as polyisobutylene), antioxidants (such as dibutylhydroxytoluene), water-absorbing polymers (such as Sumikagel SP-520 (trade name), Aquakeep 10SH (trade name), Arasorb 800F (trade name) and Sunwet 1M-1000MPS (trade name)), inorganic fillers (such as zinc oxide, calcium carbonate, titanium dioxide and silica), solubilizing aids (such as glycerol fatty acid ester [e.g., Excel (trade name)] and Crotamiton), absorption accelerators (such as fat alcohol [e.g., Kalcohl (trade name)] and moisturizers (such as triethyl citrate, polyethylene glycol and glycerol) may be contained therein appropriately.

Now, a method for the manufacture of the percutaneously absorbable preparation of this invention will be mentioned as follows. Thus, the percutaneously absorbable preparation may be manufactured, for example, in such a manner that all of ingredients for the substrate composition except pharmaceutical ingredient and hexylene glycol are dissolved by heating, then the pharmaceutical ingredient and hexylene glycol are added thereto followed by homogeneously mixing, the mixture is spread over the above-mentioned support followed, if necessary, by covering with a liner and then it is cut into a desired shape to give a product. Alternatively, after spreading over a film which has been subjected to a releasable treatment, it is transferred with pressure onto a suitable support to give a product. It is also possible that all of the ingredients are dissolved in an organic solvent such as hexane, toluene or ethyl acetate, the solution is spread over the above support, the organic solvent is removed therefrom and the residue is covered with a liner and cut into a desired shape to give a product or, alternatively, after spreading over a film which has been subjected to a releasable treatment, it is transferred with pressure onto a suitable support to give a product.

The percutaneously absorbable preparation, preferably the percutaneously absorbable patch, of this invention has an advantage that solubility, stability and skin permeability of the pharmaceutical ingredient are enhanced and good cohesive force and stabilized physical property of the substrate composition are resulted.

The percutaneously absorbable preparation of this invention has a high degree of freedom in the composition of the pharmaceuticals and, accordingly, there is a big merit for an appropriate design of the efficacy.

### Examples

The percutaneously absorbable patch of this invention will be described in more detail by means of the following Examples and Test Examples although this invention is not limited thereto.

All of the amounts in the Examples and the Test Examples are in terms of % by weight.

### Example 1

| | |
|---|---|
| Styrene-isoprene-styrene block copolymer | 10 |
| Liquid paraffin | 60 |
| Tackifying agent (Alicyclic saturated hydrocarbon resin; trade name: Arkon P-100) | 20 |
| Polyisobutylene | 7.4 |
| Hexylene glycol | 1 |
| Dibutyl hydroxytoluene | 1 |
| Estradiol | 0.1 |
| Norethisterone acetate | 0.5 |

The above-mentioned manufacturing method was applied for this formulation followed by cutting into a desired size to give a mixed preparation of estradiol and norethisterone acetate.

### Example 2

| | |
|---|---|
| Styrene-isoprene-styrene block copolymer | 30 |
| Liquid paraffin | 10 |
| Tackifying agent (Rosin ester; trade name: KE-311) | 24 |
| Polyisobutylene | 10 |
| Hexylene glycol | 10 |
| Dibutyl hydroxytoluene | 1 |
| Estradiol | 5 |
| Norethisterone acetate | 10 |

The above-mentioned manufacturing method was applied for this formulation followed by cutting into a desired size to give a mixed preparation of estradiol and norethisterone acetate.

### Example 3

| | |
|---|---|
| Styrene-isoprene-styrene block copolymer | 20 |
| Liquid paraffin | 29 |
| Tackifying agent (Hydrogenated rosin ester; trade name: Foral 105) | 30 |
| Polyisobutylene | 10 |
| Hexylene glycol | 7 |
| Dibutyl hydroxytoluene | 1 |
| Estradiol | 1 |
| Norethisterone acetate | 2 |

The above-mentioned manufacturing method was applied for this formulation followed by cutting into a desired size to give a mixed preparation of estradiol and norethisterone acetate.

### Example 4

| | |
|---|---|
| Styrene-isoprene-styrene block copolymer | 20 |
| Liquid paraffin | 35 |
| Tackifying agent (Hydrogenated rosin ester; trade name: Foral 105) | 30 |
| Polyisobutylene | 10 |
| Hexylene glycol | 1 |
| Dibutyl hydroxytoluene | 1 |
| Estradiol | 1 |
| Norethisterone acetate | 2 |

The above-mentioned manufacturing method was applied for this formulation followed by cutting into a desired size to give a mixed preparation of estradiol and norethisterone acetate.

### Example 5

| | |
|---|---|
| Styrene-isoprene-styrene block copolymer | 20 |
| Liquid paraffin | 24 |
| Tackifying agent (Hydrogenated rosin ester; trade name: Foral 105) | 30 |
| Polyisobutylene | 12 |
| Hexylene glycol | 10 |
| Dibutyl hydroxytoluene | 1 |
| Estradiol | 1 |
| Norethisterone acetate | 2 |

The above-mentioned manufacturing method was applied for this formulation followed by cutting into a desired size to give a mixed preparation of estradiol and norethisterone acetate.

### Example 6

| | |
|---|---|
| Styrene-isoprene-styrene block copolymer | 20 |
| Liquid paraffin | 21 |
| Tackifying agent (Hydrogenated rosin ester; trade name: Foral 105) | 25 |
| Polyisobutylene | 10 |
| Hexylene glycol | 8 |
| Dibutyl hydroxytoluene | 1 |
| Estradiol | 5 |
| Norethisterone acetate | 10 |

The above-mentioned manufacturing method was applied for this formulation followed by cutting into a desired size to give a mixed preparation of estradiol and norethisterone acetate.

### Example 7

| | |
|---|---|
| Styrene-isoprene-styrene block copolymer | 15 |
| Liquid paraffin | 15 |
| Tackifying agent (Hydrogenated rosin ester; trade name: Foral 105) | 60 |
| Polyisobutylene | 7.4 |
| Hexylene glycol | 1 |
| Dibutyl hydroxytoluene | 1 |
| Estradiol | 0.1 |
| Norethisterone acetate | 0.5 |

The above-mentioned manufacturing method was applied for this formulation followed by cutting into a desired size to give a mixed preparation of estradiol and norethisterone acetate.

### Example 8

| | |
|---|---|
| Styrene-isoprene-styrene block copolymer | 20 |
| Liquid paraffin | 30 |
| Tackifying agent (Hydrogenated rosin ester; trade name: Foral 105) | 30 |
| Polyisobutylene | 10 |
| Hexylene glycol | 7 |
| Dibutyl hydroxytoluene | 1 |
| Oxybutynin | 2 |

The above-mentioned manufacturing method was applied for this formulation followed by cutting into a desired size to give a preparation of oxybutynin.

### Example 9

| | |
|---|---|
| Styrene-isoprene-styrene block copolymer | 20 |
| Liquid paraffin | 35 |
| Tackifying agent (Hydrogenated rosin ester; trade name: Foral 105) | 30 |
| Polyisobutylene | 10 |
| Hexylene glycol | 1 |
| Dibutyl hydroxytoluene | 1 |
| Tizanidine | 3 |

The above-mentioned manufacturing method was applied for this formulation followed by cutting into a desired size to give a preparation of tizanidine.

### Example 10

| | |
|---|---|
| Styrene-isoprene-styrene block copolymer | 20 |
| Liquid paraffin | 24 |
| Tackifying agent (Hydrogenated rosin ester; trade name: Foral 105) | 30 |
| Polyisobutylene | 12 |
| Hexylene glycol | 10 |
| Dibutyl hydroxytoluene | 1 |
| Ketoprofen | 3 |

The above-mentioned manufacturing method was applied for this formulation followed by cutting into a desired size to give a preparation of ketoprofen.

### Example 11

| | |
|---|---|
| Styrene-isoprene-styrene block copolymer | 20 |
| Liquid paraffin | 28 |
| Tackifying agent (Hydrogenated rosin ester; trade name: Foral 105) | 25 |
| Polyisobutylene | 15 |
| Hexylene glycol | 8 |
| Dibutyl hydroxytoluene | 1 |
| Granisetron | 3 |

The above-mentioned manufacturing method was applied for this formulation followed by cutting into a desired size to give a preparation of granisetron.

### Example 12

| | |
|---|---|
| Styrene-isoprene-styrene block copolymer | 15 |
| Liquid paraffin | 15 |
| Tackifying agent (Hydrogenated rosin ester; trade name: Foral 105) | 58.6 |
| Polyisobutylene | 7.4 |
| Hexylene glycol | 1 |
| Dibutyl hydroxytoluene | 1 |
| Tulobuterol | 2 |

The above-mentioned manufacturing method was applied for this formulation followed by cutting into a desired size to give a preparation of tulobuterol.

### Comparative Example 1 (No hexylene glycol compounded)

| | |
|---|---|
| Styrene-isoprene-styrene block copolymer | 20 |
| Liquid paraffin | 36 |
| Tackifying agent (Hydrogenated rosin ester; trade name: Foral 105) | 30 |
| Polyisobutylene | 10 |
| Dibutyl hydroxytoluene | 1 |
| Estradiol | 1 |
| Norethisterone acetate | 2 |

The above-mentioned manufacturing method was applied for this formulation followed by cutting into a desired size to give a mixed preparation of estradiol and norethisterone acetate.

### Comparative Example 2 (Crotamiton used as a solubilizer)

| | |
|---|---|
| Styrene-isoprene-styrene block copolymer | 20 |
| Liquid paraffin | 29 |
| Tackifying agent (Hydrogenated rosin ester; trade name: Foral 105) | 30 |
| Polyisobutylene | 10 |
| Crotamiton | 7 |
| Dibutyl hydroxytoluene | 1 |
| Estradiol | 1 |
| Norethisterone acetate | 2 |

The above-mentioned manufacturing method was applied for this formulation followed by cutting into a desired size to give a mixed preparation of estradiol and norethisterone acetate.

### Comparative Example 3 (Acrylic substrate composition)

| | |
|---|---|
| TS-620 (Emulsion of copolymerized resin of methyl acrylate and 2-ethylhexyl acrylate; manufactured by Nippon Carbide) | 89 |
| Hexylene glycol | 8 |
| Estradiol | 1 |
| Norethisterone acetate | 2 |

The above-mentioned manufacturing method was applied for this formulation followed by cutting into a desired size to give a mixed preparation of estradiol and norethisterone acetate.

### Comparative Example 4

| | |
|---|---|
| Styrene-isoprene-styrene block copolymer | 5 |
| Liquid paraffin | 65 |
| Tackifying agent (Hydrogenated rosin ester; trade name: Foral 105) | 10 |
| Polyisobutylene | 3.4 |
| Hexylene glycol | 15 |
| Dibutyl hydroxytoluene | 1 |
| Estradiol | 0.1 |
| Norethisterone acetate | 0.5 |

The above-mentioned manufacturing method was applied for this formulation followed by cutting into a desired size to give a mixed preparation of estradiol and norethisterone acetate.

### Comparative Example 5

| | |
|---|---|
| Styrene-isoprene-styrene block copolymer | 35 |
| Liquid paraffin | 5 |
| Tackifying agent (Hydrogenated rosin ester; trade name: Foral 105) | 32 |
| Polyisobutylene | 3 |
| Hexylene glycol | 7 |
| Dibutyl hydroxytoluene | 1 |
| Estradiol | 6 |
| Norethisterone acetate | 11 |

The above-mentioned manufacturing method was applied for this formulation followed by cutting into a desired size to give a mixed preparation of estradiol and norethisterone acetate.

### Comparative Example 6

| | |
|---|---|
| Styrene-isoprene-styrene block copolymer | 20 |
| Liquid paraffin | 29 |
| Tackifying agent (Hydrogenated rosin ester; trade name: Foral 105) | 36.5 |
| Polyisobutylene | 10 |
| Hexylene glycol | 0.5 |
| Dibutyl hydroxytoluene | 1 |
| Estradiol | 1 |
| Norethisterone acetate | 2 |

The above-mentioned manufacturing method was applied for this formulation followed by cutting into a desired size to give a mixed preparation of estradiol and norethisterone acetate.

### Test Examples

### Test Example 1 (Stability Test on Solubility of the Pharmaceutical Ingredients into the Substrate Composition)

Each of the test pieces of Examples 1, 2, 3, 4, 5, 6, and 7 and Comparative Examples 1, 4 and 6 was preserved at 50°C for three months and bleeding or separation of crystals therefrom was observed. The result is shown in Table 1. In Comparative Examples 1 and 6, separation of crystals was observed. In Comparative Example 4, bleeding of hexylene glycol was observed. In the Examples, neither separation of crystals nor bleeding was observed.

**Table 1**

| | Observation of Crystallization at 50°C for Three Months | Observation of Bleeding at 50°C for Three Months |
|---|---|---|
| Example 1 | No crystal separation | No bleeding |
| Example 2 | No crystal separation | No bleeding |
| Example 3 | No crystal separation | No bleeding |
| Example 4 | No crystal separation | No bleeding |
| Example 5 | No crystal separation | No bleeding |
| Example 6 | No crystal separation | No bleeding |
| Example 7 | No crystal separation | No bleeding |
| Comp.Ex.1 | Crystals separated out | No bleeding |
| Comp.Ex.4 | No crystal separation | Bleeding noted |
| Comp.Ex.6 | Crystals separated out | No bleeding |

### Test Example 2 (Confirmation of the Property)

Each of the test pieces of Example 3 and Comparative Examples 2 and 4 was preserved at 50°C for three months and appearance and property of each test piece were observed. The result is shown in Table 2. In Example 3, flowing-out of the substrate composition was not noted while, in Comparative Examples 2 and 4, flowing-out of the substrate was noted.

**Table 2**

| | 50°C /3 Months |
|---|---|
| Example 3 | No change |
| Comparative Example 2 | Flowing-out noted |
| Comparative Example 4 | Flowing-out noted |

### Test Example 3 (Skin Permeation Test)

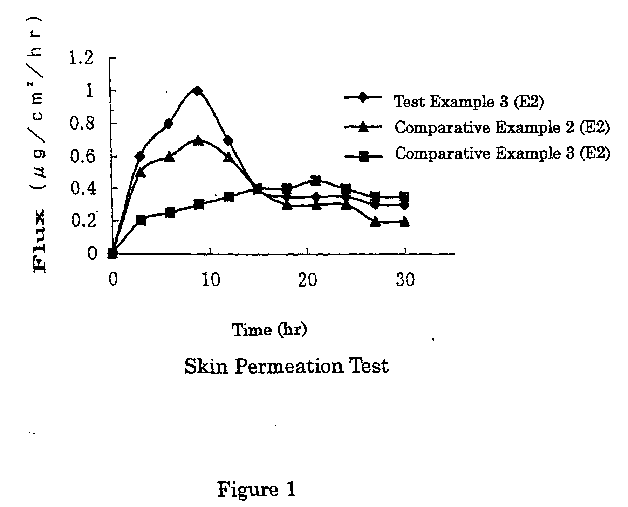

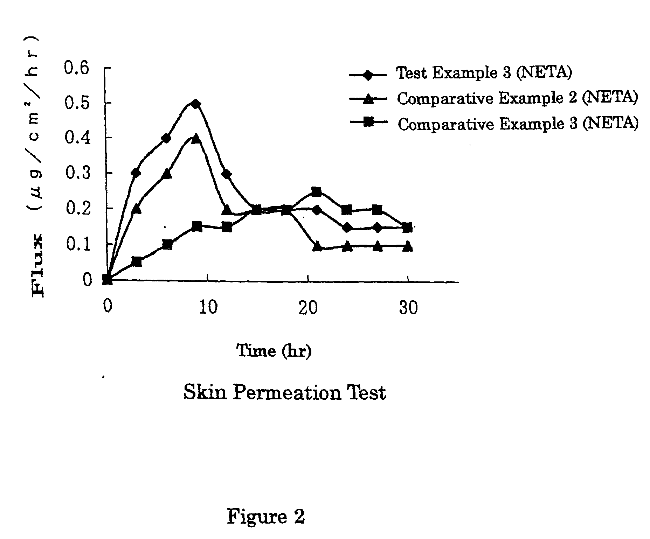

Each of the test pieces of Example 3 and Comparative Examples 2 and 3 was subjected to a permeation test (temperature: 37°C ) for the skin of back of hairless mice (six weeks age; female) using Franz-type diffusion cells. Receptor solution was collected every predetermined time after initiation of the test and a receptor solution was supplemented immediately thereafter. Permeated amount of the pharmaceutical into the receptor solution was measured by means of a high performance liquid chromatography. Sample numbers for each test piece were three. The result is shown in Fig. 1 and Fig. 2. Example 3 showed better permeability of the pharmaceutical as compared with Comparative Examples 2 and 3.

### Test Example 4 (Releasing Test)

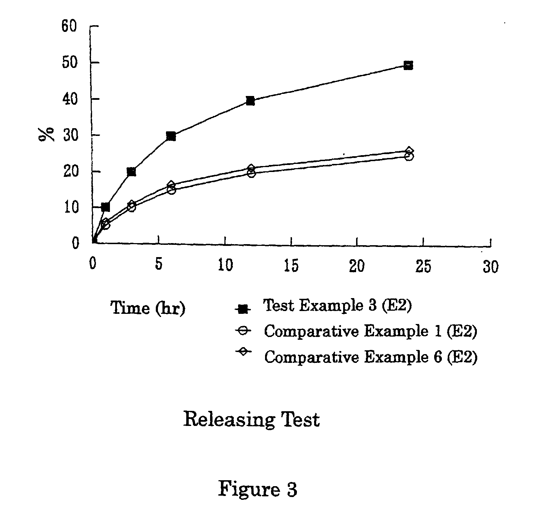

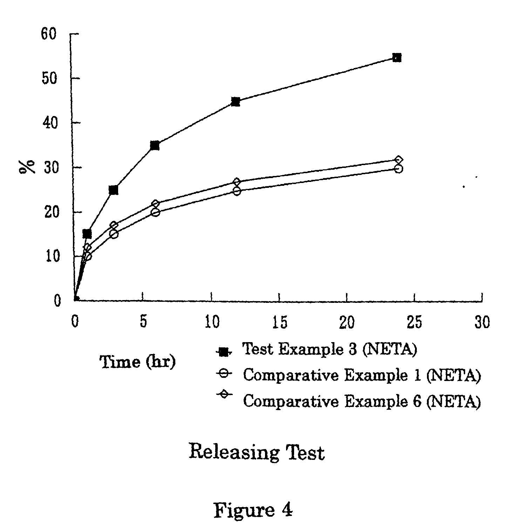

Test pieces of Example 3 and Comparative Examples 1 and 6 were subjected to a releasing test by a rotary cylinder method. The test condition was that the test solution was 900 ml, temperature of the test solution was 32.0 ± 0.5°C and the revolution of the cylinder was 50 rpm. The result is shown in Fig. 3 and Fig. 4. Good release of the pharmaceutical ingredient was achieved in Example 3 as compared with the test pieces of Comparative Examples 1 and 6.

### Test Example 5 (Adhesion Test)

Adhesion test was carried out for the test pieces of Examples 1, 2 and 3 and Comparative Examples 4 and 5 according to the following means. Thus, a test piece was applied to upper arm of ten persons (healthy and normal persons; male) for 24 hours and then its adhesive power was evaluated. The result is shown in Table 3. In the Comparative Examples, 1/2 or more exfoliation was observed in most cases while, in the Examples, no exfoliation was observed in most cases.

**Table 3**

| | No exfoliation | Edge exfoliation | 1/4 exfoliation | 1/2 exfoliation | 3/4 exfoliation | Fallen Off | Total |
|---|---|---|---|---|---|---|---|
| Example 1 | 9 | - | 1 | - | - | - | 10 |
| Example 2 | 8 | 1 | 1 | - | - | - | 10 |
| Example 3 | 9 | 1 | - | - | - | - | 10 |
| Comp.Ex.4 | - | - | - | 1 | 3 | 6 | 10 |
| Comp.Ex.5 | - | - | 1 | 7 | 1 | 1 | 10 |

## Claims

1. A substrate composition for percutaneous absorption containing a styrene-isoprene-styrene block copolymer, a softener, a tackifying resin and hexylene glycol.

2. The substrate composition for percutaneous absorption according to claim 1, wherein the content of hexylene glycol is 1-10% by weight.

3. The substrate composition for percutaneous absorption according to claim 1 or 2, wherein 10-30% by weight of a styrene-isoprene-styrene block copolymer, 10-60% by weight of a softener, 20-60% by weight of a tackifying resin and 1-10% by weight of hexylene glycol are contained to the total amount of the preparation.

4. The substrate composition for percutaneous absorption according to any of claims 1-3, wherein hexylene glycol is an absorption accelerator and/or a solubilizer for a pharmaceutical.

5. A percutaneously absorbable preparation where a pharmaceutical is contained in the substrate composition for percutaneous absorption according to any of claims 1-4.

6. The percutaneously absorbable preparation according to claim 5, wherein 0.1-10% by weight of a pharmaceutical is compounded.

7. The percutaneously absorbable preparation according to claim 5 or 6, wherein the pharmaceutical is follicular hormone and/or corpus luteum hormone.

8. The percutaneously absorbable preparation according to claim 7, wherein the follicular hormone is estradiol or a derivative thereof and its compounding amount is 0.1-5% by weight.

9. The percutaneously absorbable preparation according to claim 7, wherein the corpus luteum hormone is norethisterone, norethisterone acetate or a derivative thereof and its compounding amount is 0.5-10% by weight.
